# DEMANDE DE BREVET EUROPEEN

(11) **EP 1 813 335 A2**
(43) Date de publication de la demande: **01.08.2007**
(21) Numéro de dépôt: 07300745.2
(22) Date de dépôt: 25.01.2007
(51) Int. Cl.: B01D 11/02, A45D 40/00, A61K 8/02, B65D 85/804

(54) **Procédé de préparation d'une composition cosmétique, appareil et recharge pour la préparation d'une telle composition**

(30) Priorité: 27.01.2006 FR 0650306
(71) Demandeur: L'ORÉAL, 75008 Paris (FR)
(72) Inventeur: De la Mettrie, Roland, 78110 Le Vesinet (FR); Grollier, Jean-François, 75006 Paris (FR); Cotteret, Jean, 78480 Verneuil sur Seine (FR)
(74) Mandataire: Tanty, François

(57) **Abrégé**

La présente invention concerne un procédé de préparation d'une composition cosmétique, comportant les étapes consistant à :
- introduire dans un appareil permettant d'injecter au moins un fluide, au moins une recharge comportant dans au moins une enceinte une pluralité de produits,
- soumettre la pluralité de produits à des conditions d'injection du fluide et/ou à une action extérieure sélectionnées de manière à effectuer une extraction différenciée de l'un des produits au moins.

## Description

La présente invention concerne la préparation de compositions à appliquer sur les matières kératiniques par injection d'au moins un fluide dans une enceinte comportant au moins un produit susceptible d'être extrait de l'enceinte par la circulation du fluide dans celle-ci.

Les demandes de brevet européen EP 1 563 827-A2, EP 1 566 164-A1, EP 1 563 885-A1, EP 1 559 400-A1, EP 1 559 398-A1, EP 1 563 826-A1, EP 1 559 414-A1, EP 1 563 826-A1, EP 1 559 401-A1, EP 1 559 392-A1, EP 1 559 396-A1 et EP 1 566 163-A1 divulguent des procédés de préparation d'une composition cosmétique par percolation d'un fluide.

La demande WO 00/56629 divulgue une cartouche pour appareil d'extraction par percolation.

La demande de brevet européen EP 1 559 396 divulgue un procédé de préparation d'une composition cosmétique par percolation d'un fluide tel que de l'eau par exemple, dans une enceinte définie par une capsule similaire à celle utilisée dans les machines à café du type expresso.

Il existe un besoin pour faciliter la préparation de compositions cosmétiques personnalisées, présentant par exemple une ou plusieurs caractéristiques choisies par l'utilisateur.

Il existe encore un besoin pour faciliter la préparation à partir d'un même appareil d'extraction, par injection de fluide, d'un grand nombre de compositions à appliquer sur les matières kératiniques.

Selon l'un de ses aspects parmi d'autres, l'invention a pour objet un procédé de préparation d'une composition cosmétique, comportant les étapes consistant à :
- introduire dans un appareil d'extraction permettant d'injecter au moins un fluide, au moins une recharge comportant dans au moins une enceinte une pluralité de produits,
- soumettre la pluralité de produits à des conditions d'injection du fluide et/ou à une action extérieure sélectionnées de manière à effectuer une extraction différenciée de l'un des produits au moins.

Les paramètres des conditions d'injection sur lesquels il est possible de jouer en fonction de la composition que l'on cherche à obtenir peuvent comporter notamment la température, la pression, la durée d'injection et la nature du fluide injecté.

Grâce à l'invention, il est possible de préparer à partir d'une même recharge au moins deux compositions présentant des caractéristiques différentes, par exemple des couleurs ou des pouvoirs colorants différents, ce qui facilite l'obtention d'une composition cosmétique personnalisée.

Dans un exemple de mise en oeuvre de l'invention, l'enceinte comporte au moins deux produits ayant des températures d'extraction différentes.

L'enceinte peut comporter par exemple au moins un premier et un deuxième produit ayant des facteurs de solubilité suffisamment différents à une première température pour que l'injection du fluide à cette température provoque majoritairement l'extraction du premier produit et des facteurs de solubilité plus proches à une deuxième température, de telle sorte que l'injection du fluide à cette température provoque l'extraction du premier et du deuxième produit dans des proportions relatives différentes de celles observées à la première température.

Dans un exemple de mise en oeuvre de l'invention, la recharge comporte deux produits pouvant passer en solution sensiblement à partir de températures respectives d'injection T₁ et T₂, voire au moins trois produits correspondant à des températures T₁, T₂ et T₃.

Pour une température d'injection supérieure à T₁ mais inférieure à T₂, seul l'un des produits passe en solution.

Pour une température comprise entre T₂ et T₃, deux produits passent en solution et pour une température supérieure à T₃, tous les produits passent en solution.

Les produits contenus dans l'enceinte peuvent ainsi être extraits de façon différenciée en fonction de la température du fluide injecté, celle-ci étant sélectionnée en fonction du ou des produits que l'on souhaite extraire.

Les produits contenus dans l'enceinte peuvent encore être extraits de façon différenciée en fonction de la pression du fluide injecté, celle-ci pouvant être modifiée de façon à privilégier l'extraction d'un produit plutôt qu'un autre, par exemple.

L'enceinte peut comporter deux produits dont l'un au moins est encapsulé dans une enveloppe sensible à la température et/ou à la pression ou contenu dans une matrice polymérique sensible à la température et/ou à la pression.

L'enceinte peut encore comporter des premier et deuxième produits ayant des cinétiques d'extraction différentes pour les mêmes conditions d'injection. En sélectionnant la durée d'injection du fluide dans l'enceinte, on peut extraire le premier produit et plus ou moins du deuxième produit.

Au moins une action extérieure peut encore être exercée sur la recharge de façon préalable ou simultanée à l'injection du fluide dans l'enceinte, cette action ayant une influence sur l'extraction d'au moins un produit contenu dans l'enceinte.

L'action précitée peut comporter par exemple l'exposition de l'enceinte à un rayonnement électromagnétique ou à des vibrations ultrasonores, ce qui peut avoir un effet par exemple sur une barrière entre deux produits présents dans l'enceinte où s'effectue l'injection, sur une enveloppe encapsulant l'un des produits ou sur une matrice polymérique contenant l'un des produits.

L'exposition au rayonnement et/ou aux vibrations peut provoquer par exemple un échauffement ou autre phénomène physique permettant la rupture de la barrière ou enveloppe ou l'éclatement de la matrice polymérique.

Lorsqu'elle est préalable ou concomitante à l'injection du fluide, l'action extérieure peut être exercée par l'appareil d'extraction ou autrement, par exemple à l'aide d'un autre appareil.

L'extraction différenciée peut également être obtenue par utilisation de fluides différents ou de mélanges de fluides différents, tels que des mélanges d'eau et de solvant (par exemple du propylène glycol ou de la glycérine) à des taux variables de solvant(s). Ces fluides différents peuvent avoir un effet solubilisant différent sur les produits contenus dans l'enceinte.

Les conditions de préparation de la composition peuvent dépendre de moyens de commande qui peuvent être agencés d'une part pour recevoir au moins une information relative à une caractéristique de la composition à produire et d'autre part pour déterminer en fonction de cette information les conditions d'injection et/ou le recours à une action extérieure.

La caractéristique précitée de la composition peut être sa couleur ou son pouvoir colorant.

Ainsi, dans un exemple de mise en oeuvre de l'invention, l'enceinte comporte au moins deux produits colorés pouvant être extraits de façon différenciée, les conditions d'injection du fluide étant choisies en fonction de la teinte à obtenir.

L'invention a encore pour objet, selon un autre de ses aspects, une recharge pour un appareil de préparation d'une composition cosmétique par injection d'un fluide, comportant :
- au moins une enceinte,
- au moins deux produits cosmétiques contenus dans l'enceinte, ces produits étant extractibles de manière différenciée pour au moins deux conditions différentes d'injection du fluide dans l'enceinte et/ou d'environnement pour l'enceinte.

Les produits contenus dans l'enceinte peuvent par exemple être extraits de façon différenciée en fonction de la température du fluide et/ou de sa pression.

Au cours de l'utilisation de la recharge, l'environnement de l'enceinte peut encore être choisi de façon à exercer une action sur l'extraction des produits, par exemple par la présence d'un champ électrique ou électromagnétique ou de vibrations ultrasonores ou d'un autre stimulus.

L'enceinte peut comporter au moins deux produits colorés ayant des couleurs différentes et pouvant être extraits de façon différenciée.

L'enceinte peut comporter au moins un polymère ayant une température de changement d'état tombant dans une plage de températures d'injection pouvant être atteintes par l'appareil d'extraction. Ce polymère peut par exemple former une enveloppe encapsulant le produit ou se présenter sous la forme d'une matrice renfermant le produit.

Lorsque la température du fluide injecté dans l'enceinte est inférieure à la température de changement d'état du polymère, le produit prisonnier de l'enveloppe ou de la matrice en polymère n'est pas extrait, voire est extrait dans une moindre proportion que le ou les autres produits contenus dans l'enceinte à l'extérieur de l'enveloppe ou de la matrice.

L'enceinte peut encore comporter au moins un produit encapsulé dans au moins une capsule sensible à la pression ou contenu dans des particules poreuses.

L'enceinte peut par exemple comporter deux produits contenus dans des particules ayant des porosités différentes, de telle sorte qu'une extraction différenciée en fonction de la pression d'injection du fluide puisse avoir lieu.

L'un au moins des produits contenus dans l'enceinte peut être pulvérulent.

Les produits contenus dans l'enceinte peuvent être mélangés uniformément. Au moins deux produits contenus dans l'enceinte peuvent encore être disposés dans au moins deux régions distinctes de l'enceinte.

L'enceinte peut comporter au moins une barrière séparant deux produits différents, cette barrière présentant par exemple au moins deux états de perméabilité au fluide différents qui dépendent des conditions d'injection de celui-ci ou de l'environnement de l'enceinte.

La barrière peut être thermosensible et passer d'un état où elle est sensiblement imperméable au fluide à un état où elle est perméable au fluide, à partir d'une température tombant dans une plage de températures d'injection de l'appareil.

L'invention a encore pour objet, selon un autre de ses aspects, un appareil pour la préparation d'une composition à appliquer sur les matières kératiniques, par injection d'un fluide dans au moins une enceinte comportant au moins un produit cosmétique, comportant un système de sélection permettant de sélectionner au moins une condition d'injection du fluide dans l'enceinte parmi plusieurs.

Le système de sélection peut permettre, par exemple, de sélectionner une température d'injection parmi plusieurs.

Le système de sélection peut encore permettre, par exemple, de sélectionner une pression d'injection et/ou une durée d'injection parmi plusieurs.

Le système de sélection peut comporter un organe de commande sur lequel l'utilisateur peut agir manuellement, afin par exemple de modifier la durée et/ou la température et/ou la pression d'injection.

Le système de sélection peut être agencé pour recevoir au moins une information relative à une caractéristique de la composition à obtenir, par exemple sa couleur, et comporter un processeur permettant de déterminer de façon automatique au moins une condition d'injection du fluide en fonction de cette information.

Le système de sélection peut encore être agencé pour recevoir au moins une information relative à un individu à traiter avec la composition, par exemple relative à une couleur de peau ou de cheveux, et comporter un processeur agencé pour déterminer au moins une condition d'injection du fluide en fonction de cette information.

Le système de sélection peut encore être agencé pour recevoir au moins une information relative à la nature d'au moins un produit contenu dans l'enceinte, par exemple la quantité extraite en fonction de plusieurs valeurs d'un paramètre d'injection, et comporter un processeur permettant de déterminer au moins une condition d'injection en fonction de cette information.

L'appareil peut ainsi comporter un système de lecture d'au moins une information portée par une recharge définissant l'enceinte.

Il s'agit par exemple d'un système de lecture optique et la recharge peut comporter au moins une information lisible par le système de lecture, par exemple un code à barres.

L'information peut renseigner par exemple sur des propriétés de la solution résultant de l'extraction en fonction de conditions d'injection, par exemple une teinte en fonction de la température et/ou de la pression d'injection et/ou de la durée d'injection.

Cela peut permettre à l'appareil de déterminer précisément les conditions d'injection permettant d'obtenir un résultat souhaité.

Une information figurant sur la recharge peut encore être communiquée autrement à l'appareil d'extraction ou à un système informatique échangeant des informations avec celui-ci, par exemple en étant entrée sur un clavier ou au moyen d'une puce électronique.

L'appareil d'extraction peut comporter un injecteur agencé pour traverser une paroi d'une recharge définissant l'enceinte.

L'appareil peut comporter une pluralité d'injecteurs permettant d'injecter le fluide ou les fluides dans une enceinte ou une pluralité d'enceintes.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique, en perspective, un exemple de recharge,
- la figure 2 est une coupe axiale, schématique, de la recharge de la figure 1,
- la figure 3 représente, de manière schématique et en perspective, un exemple d'appareil d'extraction,
- la figure 4 représente de manière schématique et simplifiée un exemple de circuit d'alimentation de l'appareil d'extraction de la figure 3,
- la figure 5 est une coupe axiale, partielle, de la recharge en place dans l'appareil,
- la figure 6 est une vue analogue à la figure 3 d'une variante de réalisation de l'appareil d'extraction,
- la figure 7 représente de manière partielle et schématique une variante de circuit d'alimentation, et
- les figures 8 et 9 sont des vues analogues à la figure 1 de variantes de réalisation de la recharge et illustrent le positionnement correspondant des injecteurs.

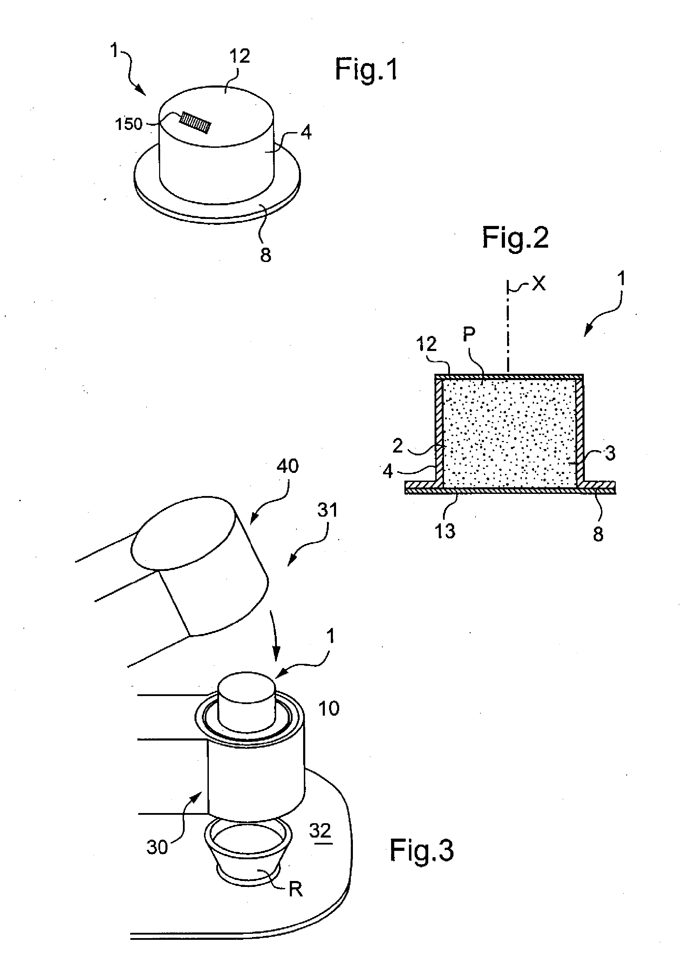

On a représenté aux figures 1 et 2 une recharge 1 contenant au moins deux produits cosmétiques P₁ et P₂ convenant à une extraction différenciée.

La recharge 1 comporte un corps tubulaire 4 et des première et deuxième parois 12 et 13 définissant une enceinte 2 contenant les produits.

Dans l'exemple illustré, la première paroi 12 est réalisée d'une seule pièce avec le corps 4, par exemple par moulage de matière plastique ou emboutissage d'un matériau en feuille comportant par exemple une couche de métal.

La deuxième paroi 13 est par exemple un film qui peut comporter au moins une couche d'un métal et qui est fixé, par exemple thermosoudé, sur une collerette 8 réalisée d'une seule pièce avec le corps 4.

L'homme du métier pourra utilement se référer pour la fabrication de la recharge 1 aux recharges existant pour la préparation de boissons par percolation, de type « expresso ».

Les produits P₁ et P₂ peuvent être extraits par circulation d'un fluide dans l'enceinte 2, la concentration en chaque produit dans la solution quittant l'enceinte étant par exemple fonction des conditions d'injection, comme cela sera précisé plus loin.

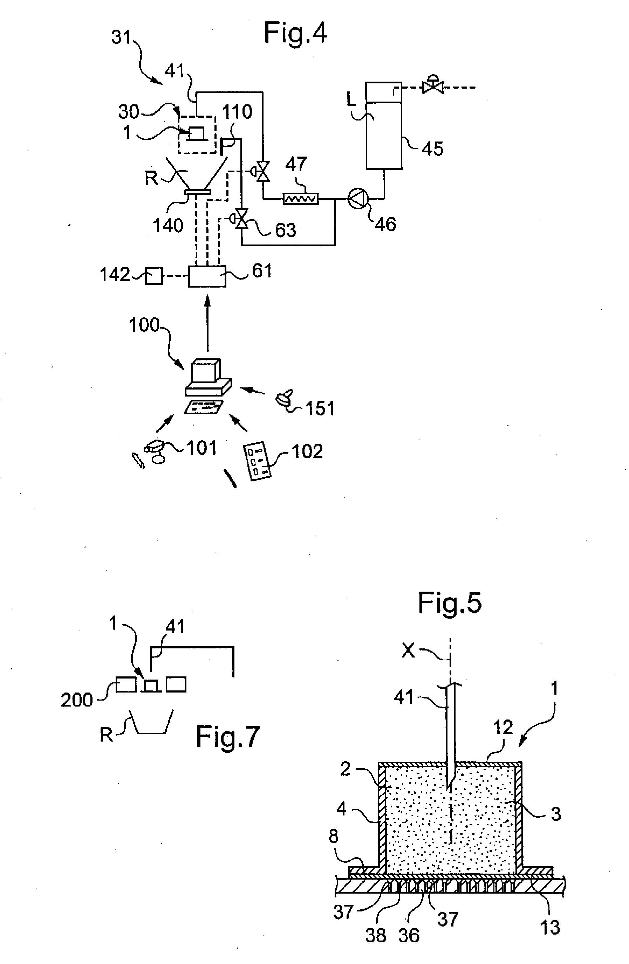

Pour utiliser la recharge 1 de la figure 1, l'utilisateur peut la placer sur un système de support 30 d'un appareil d'extraction 31 comportant également un système d'injection 40, tel qu'illustré aux figures 3 et 4.

L'appareil d'extraction 31 comporte dans l'exemple considéré un espace 32 sous le système de support 30 pour accueillir un récipient R permettant de recueillir une solution provenant de la percolation d'un fluide dans l'enceinte 2.

Le système de support 30 peut comporter, comme illustré à la figure 5, une grille 36 pourvue de reliefs 37 contre lesquels la deuxième paroi 13 peut être appliquée sous la pression du fluide injecté.

La deuxième paroi 13 est agencée pour être perforée par les reliefs 37 de la grille, lors de l'injection, sous l'effet de la pression régnant dans l'enceinte, ce qui permet au fluide injecté de s'écouler par des canaux 38 de la grille 36 vers le récipient R.

L'ouverture de la deuxième paroi 13 peut avantageusement s'effectuer de façon à sensiblement retenir dans l'enceinte considérée les corps non solubilisés.

Le récipient R peut être suffisamment large pour recueillir les solutions s'écoulant de l'enceinte compte tenu de son positionnement.

Le système d'injection 40 comprend au moins un injecteur 41 capable de franchir la première paroi 12 et de pénétrer dans l'enceinte sélectionnée.

L'appareil d'extraction 31 peut comporter des moyens d'étanchéité non représentés permettant de garantir, si nécessaire, une pénétration étanche de l'injecteur 41 dans l'enceinte sélectionnée.

Ces moyens d'étanchéité comportent par exemple au moins un joint d'étanchéité s'appliquant extérieurement sur la recharge, au moins lors de l'injection.

L'appareil d'extraction 31 peut comporter un circuit d'alimentation en fluide d'extraction ayant, comme représenté de façon schématique à la figure 4, au moins un réservoir 45 pour contenir le liquide L destiné à être injecté, une pompe 46 et un organe de chauffage 47 permettant de porter le liquide L à la température souhaitée et de créer le cas échéant de la vapeur. Celle-ci peut contribuer à générer la pression d'injection recherchée.

Dans l'exemple considéré, la pression (relative) d'injection du fluide peut atteindre par exemple au moins 3 bars (3.10⁵ Pa), voire au moins 10 bars, et la température d'injection va par exemple de 20° C à au moins 80° C, notamment de 20° C à 110° C. La température du fluide peut notamment être suffisante pour que le fluide présente des phases liquides et gazeuses lorsqu'il est injecté dans l'enceinte.

Le réservoir 45 de l'appareil d'extraction peut être rempli manuellement ou de façon automatique en étant relié à une conduite d'eau, par exemple par l'intermédiaire d'une électrovanne.

Le réservoir 45 peut être amovible, le cas échéant, afin de faciliter son remplissage manuel.

L'organe de chauffage 47 peut comporter une résistance électrique.

Un système de contrôle 61 qui peut comporter un processeur peut agir sur l'organe de chauffage 47 pour régler la température du fluide à une valeur prédéfinie.

Le système de contrôle 61 peut encore, éventuellement, agir sur la pompe 46 ou sur un circuit de contournement non représenté, de façon à régler la pression d'injection à une valeur prédéfinie.

L'appareil d'extraction 31 peut comporter en outre un capteur de niveau, utile par exemple pour détecter le besoin de remplissage du réservoir 45 ou permettre le remplissage du récipient R avec une quantité totale de composition prédéfinie.

L'appareil d'extraction peut comporter une sortie 110 permettant de distribuer dans le récipient R le liquide L sans contact avec le ou les produits contenus dans la recharge.

L'alimentation de la sortie 110 peut se faire par le biais d'une électrovanne 63 ou de tout autre moyen de contrôle de la quantité de fluide délivrée, telle que par exemple une pompe.

L'électrovanne 63 peut être contrôlée par le système de contrôle 61 de façon par exemple à avoir un volume final de composition dans le récipient correspondant à une valeur prédéfinie.

Il peut être utile de pouvoir délivrer le fluide sans passer par l'enceinte afin d'avoir un volume de composition prédéfini lorsque la durée d'injection est une condition d'injection sur laquelle on joue pour faire varier les teneurs des différents produits dans la composition.

L'appareil d'extraction peut comporter un capteur de poids 140 placé sous le récipient R, utile pour déterminer la quantité de fluide délivrée dans celui-ci. Ce capteur de poids 140 peut être relié au système de contrôle 61.

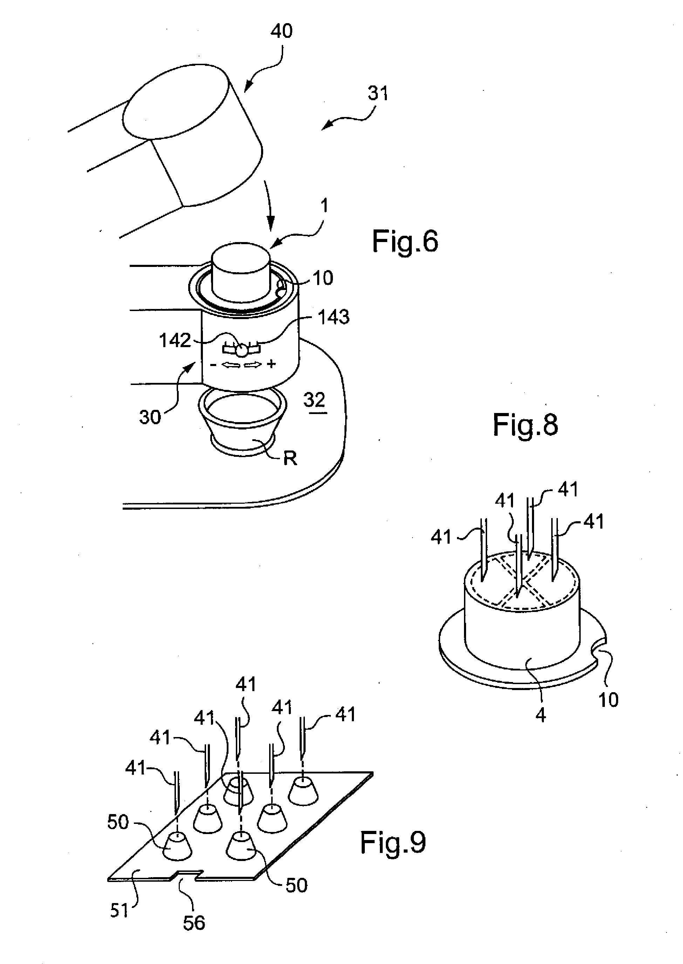

L'appareil d'extraction peut comporter, comme illustré à la figure 6, un organe de commande 142 permettant de modifier au moins une condition d'injection du fluide dans l'enceinte. Cet organe de commande 142 comporte par exemple un curseur à déplacer en fonction de la température d'injection souhaitée. L'appareil d'extraction peut comporter des graduations 143 permettant de régler la température.

On a illustré à la figure 4 la possibilité pour le circuit d'alimentation d'échanger des informations avec un système informatique 100 qui comporte par exemple au moins un micro-ordinateur, assistant personnel numérique, terminal relié à un réseau ou téléphone portable, voire qui est intégré à l'appareil d'extraction et en constitue par exemple au moins partiellement le système de contrôle 61.

Ce système informatique 100 peut être associé à un système d'acquisition d'informations comportant par exemple une caméra 101 ou tout autre dispositif d'analyse des matières kératiniques, par exemple de la couleur de la peau ou des cheveux.

Le système informatique 100 peut également être associé à un nuancier ou méchier 102 ou autre outil d'évaluation, par exemple un questionnaire.

Le système informatique 100 peut être agencé de manière à permettre à un utilisateur de sélectionner au moins une caractéristique de la composition cosmétique à préparer. Cette caractéristique est par exemple une couleur.

Le système informatique 100 peut être utilisé pour permettre à l'utilisateur de sélectionner une teinte souhaitée et le système informatique 100 peut être agencé pour adresser au système de contrôle 61 des informations permettant à ce dernier de sélectionner les conditions d'injection du fluide de façon à obtenir une composition ayant les propriétés recherchées.

Le système informatique 100 peut encore être agencé pour lire au moins une information associée à la recharge 1, cette information pouvant comporter un identifiant.

Par exemple, la recharge 1 peut comporter un code à barres 150 et le système informatique un lecteur 151 adapté à lire ce code à barres 150. Ce dernier peut contenir par exemple au moins une information relative aux produits présents dans l'enceinte. Une telle information peut permettre par exemple à l'appareil d'extraction ou au système informatique associé au système d'extraction de connaître l'évolution de la concentration en chaque produit extrait en fonction de la quantité de fluide injectée et des conditions d'injection. Cela peut faciliter le choix des conditions d'injection et le calcul des quantités de fluide à délivrer dans l'enceinte pour obtenir la composition recherchée.

L'homme du métier pourra se référer utilement, le cas échéant, aux appareils d'extraction utilisés pour préparer des boissons de type « expresso », dont les circuits d'alimentation sont susceptibles d'être au moins partiellement reproduits.

De tels appareils sont divulgués par exemple dans les publications AT 168405, US 2 688 911, DE 3 243 870, IT 1265636 et WO 2004/006740-A2.

L'appareil d'extraction peut comporter, comme illustré à la figure 7, un générateur 200 permettant d'exercer une action sur le contenu de l'enceinte.

Le générateur 200 est par exemple un générateur de rayonnement électromagnétique, par exemple de micro-ondes, ou un générateur d'ultrasons.

L'activation du générateur peut se faire manuellement ou être commandée par les moyens de contrôle 61.

Cette activation peut être préalable à l'injection du fluide ou simultanée, et avoir un effet sur l'extraction d'au moins un produit par le fluide.

L'appareil d'extraction peut comporter, comme illustré aux figures 8 et 9, une pluralité d'injecteurs 41 associés respectivement à une pluralité d'enceintes.

L'injection peut se faire dans une enceinte sélectionnée en faisant pénétrer l'injecteur dans l'enceinte correspondante, par exemple par un mouvement de l'injecteur et/ou de la capsule, ce mouvement étant par exemple provoqué par l'action de l'utilisateur sur l'un au moins du système d'injection et du système de support. Ce mouvement peut encore résulter d'un entraînement motorisé, pneumatique ou hydraulique, étant par exemple assisté par la pression du fluide.

La quantité de fluide injectée dans la ou les enceintes sélectionnées peut être prédéfinie ou choisie en fonction par exemple de la concentration recherchée pour le ou les composés extraits.

Dans l'exemple de la figure 8, la recharge 1 se présente sous la forme d'une cartouche, les enceintes étant formées par des compartiments de celle-ci, séparés par des cloisons réalisées par exemple par moulage de matière d'une seule pièce avec le corps de la recharge.

La collerette 8 peut être munie d'un détrompeur 10 se présentant par exemple sous la forme d'un relief en creux ou en saillie, par exemple une encoche.

En l'absence de détrompeur 10, le corps 4 peut être réalisé, par exemple, avec une forme non symétrique de révolution, de façon à ne permettre son positionnement dans l'appareil d'extraction associé que d'une seule manière.

Le volume de chacune des enceintes est de préférence inférieur ou égal à 25 cm³, voire à 10 cm³, étant par exemple compris entre 1 et 5 cm³.

Si nécessaire, l'utilisateur peut effectuer plusieurs injections successives dans différentes enceintes et recueillir les solutions issues de la percolation dans un même récipient, afin de les mélanger.

La recharge 1 peut se présenter autrement que sous la forme d'une cartouche et par exemple, comme illustré à la figure 9, avec des enceintes définies par des capsules 50 portées par un élément de support 51 qui se présente par exemple sous la forme d'une plaquette.

Les capsules 50 peuvent comporter une première paroi formée par exemple par emboutissage ou thermoformage d'un matériau en feuille, par exemple de l'aluminium ou un complexe comportant de l'aluminium. Cette paroi ainsi emboutie ou thermoformée peut définir l'élément de support 51 autour des capsules 50.

Les capsules 50 peuvent être fermées par une deuxième paroi qui est par exemple un film thermoscellé sur l'élément de support 51.

L'élément de support 51 peut comporter un détrompeur 56 se présentant par exemple sous la forme d'une encoche.

L'élément de support 51 et les capsules 50 peuvent encore, le cas échéant, être réalisés séparément. L'élément de support 51 est par exemple réalisé sous la forme d'une plaquette avec des trous et les capsules sont fixées dans ces trous, en fonction par exemple des produits que l'on souhaite associer au sein d'une même recharge. La fixation des capsules dans les trous de l'élément de support peut être amovible ou non. Une fixation amovible peut permettre le cas échéant à l'utilisateur de remplacer les capsules utilisées par de nouvelles. La fixation amovible peut s'effectuer par friction et/ou encliquetage, par exemple.

Dans l'exemple de la figure 9, les capsules 50 sont disposées en rangées. Dans des variantes non illustrées, l'élément de support présente une forme annulaire ou une forme de bande.

On peut encore donner à la recharge d'autres formes encore que celles représentées, par exemple une forme de barquette, sachet ou autre. Des capsules ou sachets contenant des produits différents peuvent être contenus dans un même conditionnement.

Le remplissage des enceintes peut avoir lieu au moment de la fabrication. En variante, les enceintes peuvent être remplies en fonction de la demande d'un consommateur, par exemple.

Différents compartiments d'une même recharge peuvent être fabriqués séparément puis assemblés.

Les enceintes peuvent également être, lors de la fabrication, dans une configuration déployée et cette configuration peut être modifiée au cours de la fabrication et/ou de l'utilisation.

La circulation du fluide dans une enceinte sélectionnée peut s'effectuer verticalement ou autrement.

Dans les exemples illustrés, l'injection dans une enceinte sélectionnée s'effectue par un injecteur unique, mais en variante elle peut s'effectuer avec plusieurs injecteurs.

Comme indiqué précédemment, l'enceinte dans laquelle s'effectue l'injection peut comporter au moins un matériau sensible à la pression, contenant l'un des produits.

L'enceinte peut comporter, par exemple, au moins un produit contenu dans un matériau poreux et un autre produit contenu dans un autre matériau poreux ou libre ou contenu autrement.

Comme exemple de matériaux sensibles à la pression et susceptibles d'être présents dans l'enceinte de façon à permettre une extraction différenciée en fonction des conditions d'injection du fluide, notamment de sa pression, on peut citer :
- les microparticules de silice poreuse, ayant par exemple une taille moyenne allant de 0,5 à 20 µm et une surface spécifique allant de 50 à 1 000 m²/g, notamment de 150 à 800 m²/g, telles que divulguées dans la demande EP 1 421 931 ; à titre d'exemple de microbilles de silice poreuse, on peut citer les produits commerciaux suivants : Silica Beads SB 150 de la société Myoshi, Sunsphere H-51 de la société Asahi Glass, Sunsil 130 de la société Sunjin, Spherica P-1500 de la société Ikeda Corporation et Sylosphere de la société Fuji Silysia ;
- les particules poreuses divulguées dans la demande de brevet européen EP 1 493 433 ; de telles particules peuvent présenter un diamètre moyen inférieur ou égal à 10 µm et une surface spécifique supérieure ou égale à 1 m²/g, notamment allant de 2,5 à 1 000 m²/g ; de telles particules poreuses peuvent dériver de particules poreuses organiques choisies parmi les particules de Nylon 6, Nylon 6-6, Nylon 12 et Nylon 6-12 et les particules de polyméthacrylate de méthyle ; les particules poreuses peuvent encore dériver de particules poreuses inorganiques, notamment choisies parmi les particules de silice-alumine, d'hydroxyapathie, de dioxyde de titane ou leurs mélanges ;
- les particules poreuses telles que divulguées dans la demande de brevet français FR 2 856 921, ayant par exemple une taille moyenne allant de 1 à 25 µm et choisies par exemple parmi les particules de polymère acrylique ;
- les polymères permettant par hydrolyse une libération retardée d'un composé, tels que divulgués dans la demande internationale WO 97/25366, pouvant être préparés par une réaction de condensation entre des polyols, de préférence de diols, et des dicétènes acétals. Ces polymères peuvent se présenter sous forme de matrices capables de retenir un produit tant que les conditions de sa dégradation ne sont pas remplies ;
- les particules poreuses telles que divulguées dans la demande de brevet européen EP 0 306 236, pouvant être formées de polymères réticulés, de diamètre compris entre 10 µm et 40 µm et ayant une surface spécifique allant de 20 m²/g à 200 m²/g ; ces particules sont formées par exemple d'un copolymère de styrène et de divinyle benzène ou de méthyl méthacrylate et d'éthylène glycol diméthacrylate ou de 4-vinylpirridine et d'éthylène glycol diméthacrylate.

L'enceinte peut également comporter au moins un composé sensible à la chaleur, choisi par exemple parmi :
- les polymères thermofusibles cristallins ayant un point de fusion cristalline, mesuré par analyse entalpique différentielle, compris entre 30 °C et 80 °C, tels que divulgués dans la demande de brevet européen EP 1 174 114 A1, notamment les polymères cristallins thermofusibles constitués par
   i) 85 % à 98 % en poids de motifs hydrophobes dérivés de monomères α, β-éthyléniques à chaîne latérale n-alkyle en C₁₂₋₅₀, de préférence en C₁₄₋₂₄, formant des homopolymères cristallins et
   ii) 2 à 15 % en poids de motifs hydrophiles dérivés d'acides monocarboxyliques en C₃₋₆ α, β-insaturés, d'acides dicarboxyliques insaturés en C₄₋₆, des esters et amides à courte chaîne de ces acides monocarboxyliques ou dicarboxyliques, le méthacrylate d'hydroxyéthyle et la vinylpyrrolidone ; le polymère cristallin thermofusible peut être un copolymère statistique contenant environ 10 % en poids de motifs dérivés d'acide acrylique et environ 90 % en poids de motifs dérivés de méthacrylate d'octadécyle ;
- les polymères semi-cristallins ayant un point de fusion supérieur ou égal à 30 °C tels que divulgués dans la demande de brevet européen EP 1 543 816 A1, notamment ceux ayant un point de fusion allant de 30 °C à 80 °C, par exemple ceux présentant une masse moléculaire moyenne en poids supérieure ou égale à 1 000, par exemple allant de 5 000 à 1 000 000, voire de 10 000 à 500 000 ; de tels polymères semi-cristallins peuvent comporter
   i) un squelette polymérique et ii) au moins une chaîne latérale cristallisable et/ou une séquence organique cristallisable faisant partie du squelette du polymère cristallin ; de tels polymères semi-cristallins peuvent être choisis parmi les copolymères séquencés comportant au moins une séquence cristallisable et au moins une séquence amorphe, les homopolymères et les copolymères portant au moins une chaîne latérale cristallisable par motif de répétition, et leurs mélanges ; la séquence cristallisable peut être de nature différente de la séquence amorphe. Ces polymères semi-cristallins peuvent avoir une chaîne organique cristallisable et/ou une séquence cristallisable représentant au moins 30 % et de préférence 40 % du poids total du polymère ; ces polymères semi-cristallins peuvent être choisis parmi :
      - les copolymères séquencés de polyoléfines à cristallisation contrôlée,
      - les polycondensats de type polyesters,
      - les homo- ou co-polymères portant au moins une chaîne latérale cristallisable,
      - leurs mélanges ;
      - les polymères ayant une masse moléculaire moyenne allant de 500 à 500 000 et contenant au moins un groupement polyorganosiloxane et au moins deux groupements capables d'établir des liaisons hydrogènes, choisis parmi les esters, les amides, les sulfonamides, les carbamates, le thiocarbamate, l'urée, l'uréthane, la thio-urée, les groupes oxamido, gluanamido et biguanido et les combinaisons de ceux-ci, et au moins un composé siliconé cristallin, tels que divulgués dans la demande de brevet US 2004/0120912 A1 ;
      - les polyuréthanes polyéthers thermosensibles obtenus à partir d'un composé diisocianate et le polyoxyalkylène glycol, tels que divulgués dans la demande de brevet européen EP 0 692 506 A2, et
      - les polymères semi-cristallins solides à température ambiante ayant une température de fusion inférieure à 70 °C et comportant un squelette polymérique et au moins une chaîne organique latérale cristallisable et/ou une séquence organique cristallisable faisant partie dudit squelette polymérique, tels que divulgués dans la demande de brevet français FR 2 863 889.

### Exemple proposé

On réalise deux recharges identiques colorantes contenant dans l'enceinte une composition anhydre ayant la formulation suivante (teneurs massiques):
- colorant rouge (2Nβ hydroxy éthylamino 5 aminonitrobenzène) enrobé par un polymère thermofusible de point de fusion 80 °C 20 %
- colorant jaune (2N méthyl amino 4βγ dihydroxy propyloxy nitrobenzène) enrobé de polymère thermofusible de point de fusion compris entre 85 °C et 90 °C 30 %
- maltodextrine 50 %

On injecte dans la première recharge de l'eau à une température de 80 °C et une pression de 15 bars. On recueille 10 ml de composition permettant de teindre des cheveux humains à 90 % blancs dans une nuance rouge.

On injecte dans la deuxième recharge de l'eau à une température de 95 °C et une pression de 15 bars. On recueille 10 ml de composition permettant la teinture de cheveux humains à 90 % blancs dans une nuance orangée.

L'expression « comportant un » doit être comprise comme étant synonyme de « comportant au moins un », sauf si le contraire est spécifié.

## Revendications

1. Procédé de préparation d'une composition cosmétique, comportant les étapes consistant à :
- introduire dans un appareil permettant d'injecter au moins un fluide, au moins une recharge comportant dans au moins une enceinte une pluralité de produits,
- soumettre la pluralité de produits à des conditions d'injection du fluide et/ou à une action extérieure sélectionnées de manière à effectuer une extraction différenciée de l'un des produits au moins.

2. Procédé selon la revendication 1, dans lequel au moins deux produits contenus dans l'enceinte peuvent être extraits de façon différenciée en fonction de la température d'injection du fluide et dans lequel la température du fluide injecté dans l'enceinte est sélectionnée en fonction du ou des produits que l'on souhaite extraire et éventuellement des concentrations en ce ou ces produits que l'on souhaite obtenir.

3. Procédé selon l'une des revendications 1 et 2, dans lequel au moins deux produits contenus dans l'enceinte peuvent être extraits de façon différenciée en fonction de la pression du fluide et dans lequel la pression du fluide injecté dans l'enceinte est sélectionnée en fonction du ou des produits que l'on souhaite extraire et éventuellement des concentrations en ce ou ces produits que l'on souhaite obtenir.

4. Procédé selon l'une quelconque des revendications précédentes, dans lequel au moins une action extérieure est exercée sur la recharge de façon préalable ou simultanée à l'injection du fluide dans l'enceinte, cette action ayant une influence sur l'extraction par le fluide d'au moins un produit contenu dans l'enceinte.

5. Procédé selon la revendication 4, l'action extérieure étant exercée par l'appareil d'extraction.

6. Procédé selon l'une des revendications 4 et 5, l'action comportant l'exposition de l'enceinte à un rayonnement électromagnétique.

7. Procédé selon l'une des revendications 4 à 6, l'action comportant l'exposition de l'enceinte à des ultrasons.

8. Procédé selon l'une quelconque des revendications précédentes, l'enceinte comportant au moins deux produits colorés pouvant être extraits de façon différenciée, procédé dans lequel les conditions d'injection du fluide sont choisies en fonction d'une teinte à obtenir.

9. Procédé selon l'une des revendications 4 à 7, l'enceinte comportant au moins deux produits colorés pouvant être extraits de façon différenciée, procédé dans lequel l'action extérieure est sélectionnée en fonction d'une teinte à obtenir.

10. Procédé selon l'une quelconque des revendications précédentes, la pression du fluide étant supérieure ou égale à 1 bar, mieux 3 bars, encore mieux 10 bars.

11. Procédé selon l'une quelconque des revendications précédentes, dans lequel la ou les solutions sont recueillies dans un récipient.

12. Procédé selon l'une quelconque des revendications précédentes, le fluide étant de l'eau.

13. Recharge pour appareil d'extraction permettant la préparation d'une composition cosmétique par injection d'un fluide dans la recharge, cette dernière comportant :
- au moins une enceinte,
- au moins deux produits cosmétiques contenus dans l'enceinte, ces produits étant extractibles de manière différenciée pour au moins deux conditions différentes d'injection du fluide dans l'enceinte et/ou lorsqu'une action extérieure prédéfinie est exercée sur l'enceinte.

14. Recharge selon la revendication 13, l'enceinte comportant au moins deux produits ayant des températures d'extraction différentes.

15. Recharge selon la revendication 14, l'enceinte comportant au moins un premier produit et un deuxième produit ayant des facteurs de solubilité suffisamment différents à une première température pour que l'injection du fluide à cette première température provoque majoritairement l'extraction du premier produit et des facteurs de solubilité plus proches à une deuxième température de telle sorte que l'injection du fluide à cette deuxième température provoque l'extraction du premier produit et deuxième produit dans des proportion relatives différentes de celles observées à la première température.

16. Recharge selon l'une quelconque des revendications 13 à 15, les premier et deuxième produits ayant des couleurs différentes ou conduisant à des effets tinctoriaux différents.

17. Recharge selon l'une quelconque des revendications 13 à 16, l'enceinte comportant au moins un produit encapsulé dans un matériau sensible à la température et/ou à la pression.

18. Recharge selon l'une quelconque des revendications 13 à 17, l'enceinte comportant au moins un produit contenu dans une matrice polymérique sensible à la température et/ou à la pression.

19. Recharge selon l'une quelconque des revendications 13 à 18, l'enceinte comportant au moins un agent de coloration incorporé dans un matériau poreux, notamment dans des particules poreuses.

20. Appareil d'extraction pour la préparation d'une composition à appliquer sur les matières kératiniques, par injection d'un fluide dans au moins une enceinte comportant au moins un produit cosmétique, l'appareil comportant un système de sélection permettant de sélectionner au moins une condition d'injection du fluide dans l'enceinte parmi plusieurs.

21. Appareil selon la revendication 20, le système de sélection permettant de sélectionner la température d'injection.

22. Appareil selon la revendication 20 ou 21, le système de sélection permettant de sélectionner la pression d'injection.

23. Appareil selon l'une quelconque des revendications 20 à 22, le système de sélection étant agencé pour recevoir au moins une information relative à une caractéristique de la composition à obtenir et l'appareil comportant un processeur permettant de déterminer au moins une condition d'injection du fluide en fonction de cette information.

24. Appareil selon la revendication 23, la caractéristique de la composition étant sa couleur ou son pouvoir tinctorial.

25. Appareil selon l'une quelconque des revendications 20 à 24, le système de sélection étant agencé pour recevoir au moins une information relative à la nature d'au moins un produit contenu dans l'enceinte et l'appareil comportant un processeur permettant de déterminer au moins une condition d'injection en fonction de cette information.

26. Appareil selon l'une quelconque des revendications 20 à 25, comportant un système de lecture (151) d'au moins une information (150) portée par une recharge définissant l'enceinte.

27. Appareil selon l'une quelconque des revendications 20 à 26, comportant un générateur (200) de rayonnement électromagnétique agencé pour exposer l'enceinte à un tel rayonnement.

28. Appareil selon l'une quelconque des revendications 20 à 27, comportant un générateur d'ultrasons (200) agencé pour exposer l'enceinte à des vibrations ultrasonores.

29. Appareil selon l'une quelconque des revendications 20 à 28, le fluide injecté étant sous pression d'au moins 3 bars et à une température supérieure à 80 °C.

30. Appareil selon l'une quelconque des revendications 20 à 29, le fluide étant sous phases vapeur et liquide.

31. Appareil selon l'une quelconque des revendications 20 à 25, le fluide étant de l'eau.

32. Ensemble comportant :
- une recharge définissant au moins une enceinte comportant deux produits cosmétiques pouvant être extraits de façon différenciée selon les conditions d'injection du fluide dans l'enceinte,
- un appareil d'extraction (31) tel que défini dans l'une quelconque des revendications 20 à 30.
